(19) Europäisches Patentamt
European Patent Office
Office européen des brevets

(11) Veröffentlichungsnummer : **0 062 823**
**B1**

(12) # EUROPÄISCHE PATENTSCHRIFT

(45) Veröffentlichungstag der Patentschrift : **30.01.85**

(21) Anmeldenummer : **82102614.3**

(22) Anmeldetag : **29.03.82**

(51) Int. Cl.⁴ : **C 08 G 18/78**, C 08 G 18/76, C 08 G 18/14, C 07 C127/00, C 07 C127/24

(54) **Verfahren zur Herstellung von Harnstoff- und/oder Biuretgruppen aufweisenden aromatischen Polyisocyanaten und ihre Verwendung als Isocyanatkomponente bei der Herstellung von Polyurethankunststoffen.**

(30) Priorität : **10.04.81 DE 3114638**

(43) Veröffentlichungstag der Anmeldung : **20.10.82 Patentblatt 82/42**

(45) Bekanntmachung des Hinweises auf die Patenterteilung : **30.01.85 Patentblatt 85/05**

(84) Benannte Vertragsstaaten : **BE DE FR GB IT**

(56) Entgegenhaltungen :
**EP-B- 0 003 505**
**DE-B- 2 032 174**

(73) Patentinhaber : **BAYER AG**
**Konzernverwaltung RP Patentabteilung**
**D-5090 Leverkusen 1 Bayerwerk (DE)**

(72) Erfinder : **Scholl, Hans-Joachim, Dr.**
**Rudolf-Sohm-Strasse 28**
**D-5000 Köln 60 (DE)**
Erfinder : **Hettel, Hans, Dr.**
**Hahnenweg 5**
**D-5000 Köln 80 (DE)**

**EP 0 062 823 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von neuen Harnstoff- und/oder Biuretgruppen aufweisenden aromatischen Polyisocyanaten durch Umsetzung von Diisocyanaten mit unterschüssigen Mengen an primären und/oder sekundären Diaminen, wobei man als Reaktionspartner spezielle, nachstehend näher beschriebene Diisocyanate und/oder spezielle, nachstehend näher beschriebene Diamine einsetzt bzw. mitverwendet, sowie die Verwendung der Verfahrensprodukte als Isocyanatkomponente bei der Herstellung von Polyurethankunststoffen.

Harnstoff- und/oder Biuret-Bindungen enthaltende Polyisocyanate sind bekannt (vgl. z. B. « Polyurethanes », Chemistry and Technology, Part I, von Saunders und Frisch, Interscience Publishers, (1962), Seiten 190 ff).

Normalerweise läßt man derartige Harnstoff- und/oder Biuretbindungen durch Einwirken von Wasser auf Polyisocyanate unter Verlust wertvoller NCO-Anteile entstehen.

Darüber hinaus offenbart die GB-PS 1 078 390 die Bildung von Biureten direkt aus Diaminen und aromatischen Polyisocyanaten, indem man diese Reaktion in Lösungsmitteln, deren Siedepunkt unter dem Siedepunkt des Isocyanates liegt, wie z. B. Chloroform, durchführt. Der Nachteil dieses Verfahrens besteht darin, daß im Anschluß an die Reaktion das Lösungsmittel durch Destillation entfernt werden muß. Eine direkte Umsetzung der in den Beispielen angeführten Diamine mit den beschriebenen Isocyanaten ist ohne Lösungsmittel nicht möglich, da die sofort entstehenden schwerlöslichen Polyharnstoffe den weiteren Angriff des Isocyanates verhindern.

Das Verfahren der DE-OS 1 963 190 beschreibt auch die direkte Umsetzung von aromatischen Polyaminen mit primären Aminogruppen und Polyisocyanaten zu löslichen Biureten ohne Lösungsmittel, wenn man Polyamine mit primären Aminogruppen und abgeschwächter Nucleophilie verwendet. Die Reaktivität der Amine gegenüber den Isocyanaten muß so weit abgeschwächt sein, daß eine homogene Vermischung von Amin und Isocyanat möglich ist, bevor die Umsetzung der beiden Komponenten beginnt. Dies erfordert stark erhöhte Temperaturen mit der Gefahr unerwünschter Nebenreaktionen und der Ausfällung fester Bestandteile.

Es war daher die Aufgabe der Erfindung, ein neues Verfahren zur Herstellung von stabilen, flüssigen Harnstoff- und/oder Biuretgruppen aufweisenden aromatischen Polyisocyanaten aus Diaminen und Diisocyanaten zur Verfügung zu stellen, das auch ohne Mitverwendung von Hilfslösungsmitteln bei üblichen Herstellungstemperaturen in flüssiger Phase durchgeführt werden kann, ohne daß Ausfällungen von unlöslichen Harnstoff- und/oder Biuretanteilen beobachtet werden müssen.

Diese Aufgabe konnte durch das nachstehend näher beschriebene erfindungsgemäße Verfahren gelöst werden.

Gegenstand der Erfindung ist ein Verfahren zur Herstellung von gegebenenfalls als Homologen- und/oder Isomerengemisch vorliegenden Harnstoff- und/oder Biuretgruppen aufweisenden aromatischen Polyisocyanaten durch Umsetzung von Harnstoff- und Biuretgruppen-freien aromatischen Diisocyanaten mit Harnstoff- und Biuret-gruppen-freien Diaminen mit primären oder sekundären Aminogruppen bei 20 bis 180 °C unter Einhaltung eines Äquivalentverhältnisses zwischen Isocyanatgruppen und primären bzw. sekundären Aminogruppen von 5 : 1 bis 100 : 1, dadurch gekennzeichnet, daß mindestens 25 Mol-% der zum Einsatz gelangenden Diisocyanate der Formel

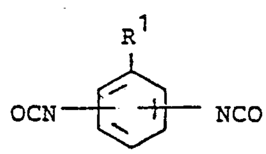

und/oder mindestens 25 Mol-% der zum Einsatz gelangenden Diamine der Formel

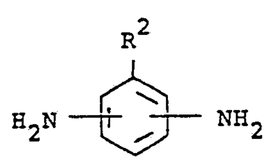

entsprechen, wobei in diesen Formeln

$R^1$ und $R^2$ für gleiche oder verschiedene Reste stehen und jeweils gesättigte aliphatische Kohlenwasserstoffreste mit 6 bis 18 Kohlenstoffatomen bedeuten.

Gegenstand der Erfindung ist auch die Verwendung der nach dem erfindungsgemäßen Verfahren erhaltenen, Harnstoff- und/oder Biuretgruppen aufweisenden aromatischen Polyisocyanate, gegebenenfalls in einer in überschüssigem Ausgangsdiisocyanat gelösten Form, als Isocyanatkomponente bei der Herstellung von Polyurethankunststoffen nach dem Isocyanat-Polyadditionsverfahren.

Beim erfindungsgemäßen Verfahren können beliebige aromatische Diisocyanate eingesetzt werden, beispielsweise solche der Formel

$$Q(NCO)_2$$

in welcher

Q einen aromatischen Kohlenwasserstoffrest mit 6 bis 15, vorzugsweise 6 bis 13 Kohlenstoffatomen,

bedeutet, wie z. B. p-Phenylendiisocyanat, 2,4- und 2,6-Diisocyanatotoluol, 2,4'- und 4,4'-Diisocyanatodiphenylmethan, 3,4'-Diisocyanato-4-methyl-diphenylmethan, 3,2'-Diisocyanato-4-methyl-diphenylmethan, 1,5-Diisocyanato-naphthalin oder 4,4'-Diisocyanato-diphenylpropan-(1,1) sowie beliebige Gemische derartiger Diisocyanate.

Beim erfindungsgemäßen Verfahren können beliebige Diamine mit primären und/oder sekundären Aminogruppen eingesetzt werden, beispielsweise solche der Formel

$$Q'(NHR^3)_2$$

in welcher

Q' für einen Rest steht, der bezüglich seiner Bedeutung der Definition von Q entspricht, und der gleich oder verschieden groß Q ist und

$R^3$ für Wasserstoff oder einen Alkylrest mit 1 bis 4 Kohlenstoffatomen steht.

Beispiele derartiger Diamine sind p-Phenylendiamin, 2,4- und 2,6-Diamino-toluol, N,N'-Dimethyl-2,4-diaminotoluol, 2,4'- und/oder 4,4'-Diamino-diphenylmethan, 3,4'-Diamino-4-methyl-diphenylmethan, 3,2'-Diamino-4-methyl-diphenylmethan oder 1,5-Diamino-naphthalin. Beim erfindungsgemäßen Verfahren können beliebige Gemische derartige Diamine eingesetzt werden.

Erfindungswesentlich ist nun, daß mindestens 25 Mol-% der beim erfindungsgemäßen einzusetzenden Diisocyanate der Formel

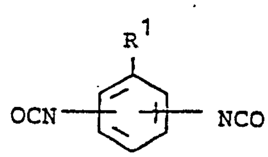

und/oder mindestens 25 Mol-% der beim erfindungsgemäßen Verfahren einzusetzenden Diamine der Formel

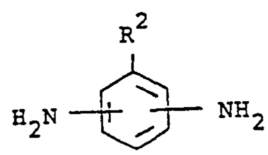

entsprechen, wobei

$R^1$ und $R^2$ die bereits oben genannte Bedeutung haben.

Besonders bevorzugt ist die Ausführungsform des erfindungsgemäßen Verfahrens gemäß welcher die beim erfindungsgemäßen Verfahren einzusetzenden Diamine ausschließlich aus Diaminen der zuletzt genannten Formel bestehen und als Diisocyanate ausschließlich Diisocyanate mit aromatisch gebundenen Isocyanatgruppen der beispielhaft genannten Art eingesetzt werden.

Besonders bevorzugt werden beim erfindungsgemäßen Verfahren solche erfindungswesentlichen Diamine der zuletzt genannten allgemeinen Formel eingesetzt, für welche $R^2$ für einen gesättigten aliphatischen Kohlenwasserstoffrest mit 8 bis 15, vorzugsweise 10 bis 13 Kohlenstoffatomen steht und die in Form von Isomeren- und/oder Homologengemischen vorliegen.

Die erfindungswesentlichen Diamine der zuletzt genannten allgemeinen Formel werden durch Dinitrierung der ihnen zugrundeliegenden Alkylbenzole und anschließende Überführung der Nitrogruppen in Aminogruppen erhalten. Die Herstellung der besonders bevorzugten Homologen- bzw. Isomerengemische erfolgt beispielsweise dergestalt, daß man als technische Homologen- bzw. Isomerengemische vorliegende Alkylbenzole, die zu mindestens 90 Gew.-%, vorzugsweise ausschließlich aus Alkylbenzolen der Formel

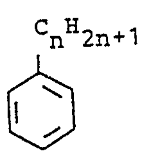

bestehen, wobei

n für eine ganze Zahl von 8 bis 15, vorzugsweise 10 bis 13 steht,

und die bei 1 013 mbar einen Siedebereich gemäß ASTM D 86 von 10-50 °C, vorzugsweise 20-30 °C innerhalb des Temperaturbereichs von 270 °C bis 330 °C aufweisen, dinitriert und anschließend in die Diamine überführt.

Die Herstellung derartiger Alkylbenzol-Gemische erfolgt in an sich bekannter Weise durch Alkylierung von benzol mit technischen Olefingemischen oder technischen Alkylchloridgemischen, wie beispielsweise in Kirk-Othmer, Enzyclopedia of Chemical Technology, third edition, Vol. 2, (1978) auf Seiten 50 bis 61 beschrieben.

Anschließend erfolgt die Überführung der Alkylbenzole in die entsprechenden Dinitroverbindungen durch eine an sich bekannte Nitrierungsreaktion, beispielsweise im Temperaturbereich von 10 bis 30 °C unter Verwendung von beispielsweise 2 bis 3 Mol konzentrierter Salpetersäure pro Mol Alkylbenzol, wobei die Salpetersäure in Form eines Gemisches mit konzentrierter Schwefelsäure (Nitriersäure) zur Anwendung gelangt. Bei den hierbei anfallenden Alkyl-substituierten Dinitrobenzolen sind gemäß Kernresonanzmessungen über 95 % aller Nitrogruppen in meta-Stellung zueinander angeordnet. Außerdem kann davon ausgegangen werden, daß ca. 10 bis 30 Gew.-% der dinitrierten Alkylbenzole 1-Alkyl-2,6-dinitrobenzole und ca. 70 bis 90 Gew.-% 1-Alkyl-2,4-dinitrobenzole darstellen. Diese Isomerenverteilung ist jedoch keineswegs wesentlich.

So wäre es auch beispielsweise möglich, für die nächste Stufe dinitrierte Alkylbenzole einzusetzen, in denen andere Mengenverhältnisse der genannten Isomeren vorliegen. So könnte beispielsweise für die nächste Stufe auch 1-Alkyl-2,4-dinitrobenzol in 2,6-freier Form eingesetzt werden, wie es beispielsweise durch zweistufige Nitrierung von Alkylbenzol über die Zwischenstufe des zunächst isolierten 1-Alkyl-4-nitrobenzols erhältlich ist.

Die nächste Stufe besteht in der an sich bekannten Hydrierung der Nitrogruppen zu den entsprechenden primären Aminogruppen beispielsweise unter Verwendung von Raney-Nickel als Hydrierungskatalysator bei beispielsweise 20 bis 60 °C und einem Wasserstoffdruck von beispielsweise 20 bis 40 bar.

Die bei dieser Hydrierung anfallenden Diamine der o. g. Formel entsprechen selbstverständlich bezüglich der Stellung der Aminogruppen den bezüglich der Stellung der Nitrogruppen gemachten Ausführungen und bezüglich der Natur des Restes R den bei der Beschreibung der zu ihrer Herstellung einzusetzenden Alkylbenzolen gemachten Ausführungen.

Bezüglich der Art der anstelle oder zusammen mit den erfindungswesentlichen Diaminen einzusetzenden Diisocyanate der Formel

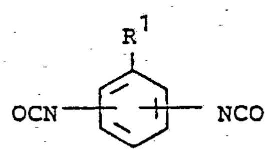

gelten alle bezüglich der Diamine gemachten Ausführungen sinngemäß. Diese Diisocyanate werden durch die an sich bekannte Phosgenierungsreaktion aus den zuletzt beschriebenen Diaminen erhalten. Hierzu wird beispielsweise das Diamin in einem Hilfslösungsmittel wie Chlorbenzol gelöst und in eine Lösung von Phosgen in Chlorbenzol unter Rühren und Kühlen bei − 20 bis + 5 °C, vorzugsweise − 10 bis 0 °C eingetropft (Kaltphosgenierung), wonach das Reaktionsgemisch unter fortlaufendem Rühren und Einleiten von Phosgen auf 80 bis 130 °C, vorzugsweise 90 bis 110 °C erhitzt wird, um das zunächst gebildete Carbamidsäurechlorid in das angestrebte Diisocyanat zu überführen (Heißphosgenierung). Anschließend wird das Reaktionsgemisch in an sich bekannter Weise aufgearbeitet. Selbstverständlich kann die Überführung der Diamine in die erfindungsgemäßen Diisocyanate auch nach beliebigen anderen, an sich bekannten Phosgeniermethoden erfolgen.

Die Durchführung des erfindungsgemäßen Verfahren erfolgt beispielsweise dergestalt, daß man die Diaminkomponente bei 20 bis 180 °C, vorzugsweise bei 30 bis 150 °C unter innigem Durchmischen in die vorgelegte Polyisocyanatkomponente eindosiert. Das Mengenverhältnis der Reaktionspartner wird dabei so gewählt, daß ein Äquivalentverhältnis zwischen Isocyanatgruppen und primären und/oder sekundären Aminogruppen von 5 : 1 bis 100 : 1, vorzugsweise 5 : 1 bis 40 : 1 vorliegt.

Gemäß bevorzugten Ausführungsformen des erfindungsgemäßen Verfahrens wird die Reaktion in Gegenwart von die Isocyanat-Additionsreaktion beschleunigenden Katalysatoren und/oder in Gegenwart von unerwünschten Nebenreaktionen wie z. B. Isocyanuratbildung verhindernden Zusatzmitteln wie z. B. geringen Mengen an Alkylierungsmitteln durchgeführt.

Geeignete Katalysatoren sind beispielsweise organische Metallverbindungen wie Zinkacetylacetonat, Kobaltacetylacetonat, Zinn-(II)-octoat oder Dibutylzinndilaurat. Bevorzugt sind die genannten Acetylacetonate. Diese Katalysatoren werden, falls überhaupt, in Mengen von 0,001 bis 2 Gew.-%, vorzugsweise von 0,005 bis 0,5 Gew.-%, bezogen auf Reaktionsgemisch, eingesetzt.

Geeignete Alkylierungsmittel sind z. B. Methyljodid, Dialkylsulfate oder aliphatische bzw. aromatische Sulfonsäurealkylester wie z. B. Toluolsulfonsäureethylester, die, falls überhaupt, in Mengen von 0,001 bis 2 Gew.-%, vorzugsweise 0,01 bis 1 Gew.-%, bezogen auf Gesamtgemisch, eingesetzt werden.

Die Frage, ob beim erfindungsgemäßen Verfahren in erster Linie Harnstoff- oder Biuretgruppen

aufweisende Polyisocyanate entstehen, hängt von der Reaktivität der Reaktionspartner, der Reaktionstemperatur, der Reaktionsdauer und der Art und Menge der gegebenenfalls eingesetzten Katalysatoren ab.

Es kann davon ausgegangen werden, daß bei unkatalysierter Reaktion unterhalb 100 °C in erster Linie Harnstoffgruppen aufweisende Polyisocyanate und oberhalb 100 °C zunehmend Biuretgruppen aufweisende Polyisocyanate entstehen, deren Bildung katalytisch beschleunigt werden kann. Selbstverständlich können bei der Durchführung des erfindungsgemäßen Verfahrens auch Gemische von Harnstoffgruppen aufweisenden Polyisocyanaten mit Biuretgruppen aufweisenden Polyisocyanaten anfallen. Da beim erfindungsgemäßen Verfahren stets ein Überschuß an Ausgangsdiisocyanat eingesetzt wird, fallen die Verfahrensprodukte in Form von Lösungen in überschüssigem, unmodifiziertem Ausgangsdiisocyanat an, die beispielsweise durch Dünnschichtdestillation von nicht-umgesetztem Ausgangsdiisocyanat befreit werden können, die jedoch vorzugsweise in Form der genannten Lösungen der erfindungsgemäßen Verwendung zugeführt werden.

Der besondere Vorteil des erfindungsgemäßen Verfahrens liegt in dem Umstand begründet, daß wegen der ausschließlichen oder teilweisen Verwendung der erfindungswesentlichen Diisocyanate bzw. Diamine keinerlei Kristallisationsprobleme auftreten. Es entstehen somit beim erfindungsgemäßen Verfahren, gleichgültig, ob die Umsetzung auf der Harnstoffstufe, beispielsweise durch Abkühlen, abgebrochen wird oder nicht, stets Feststofffreie Lösungen der erfindungsgemäßen Verfahrensprodukte im Ausgangsdiisocyanat.

Die erfindungsgemäßen Verfahrensprodukte eignen sich insbesondere in Form der beim erfindungsgemäßen Verfahren entstehenden Lösungen als Isocyanatkomponente bei der Herstellung von Polyurethankunststoffen nach dem Isocyanat-Polyadditionsverfahren, wobei es unerheblich ist, ob als Verfahrensprodukte vornehmlich Harnstoffgruppen aufweisende Polyisocyanate oder Biuretgruppen aufweisende Polyisocyanate vorliegen. Die erfindungsgemäßen Verfahrensprodukte eignen sich insbesondere zur Herstellung von Polyurethanschaumstoffen nach den an sich bekannten Methoden des Standes der Technik.

Die nachfolgenden Beispiele dienen zur weiteren Erläuterung der Erfindung. Alle Prozentangaben beziehen sich auf Gewichtsprozente.

Herstellung von Ausgangsmaterialien

Diamingemisch I :

a) Nitrierung eines Alkylbenzolgemisches :

In 1,97 kg eines Homologengemisches linearer Alkylbenzole, deren Alkylketten eine Länge von 10 bis 13 C-Atomen bei einer mittleren Kettenlänge von etwa 12 C-Atomen aufweisen und das gemäß ASTM D 86 bei 1 013 mbar zwischen 283 und 313 °C siedet, wird eine Mischung von 1 136 ml 98 %iger Salpetersäure und 1 584 ml 96 %iger Schwefelsäure unter Kühlen so eingetropft, daß die Innentemperatur bei 10 bis 15 °C liegt. Nach Beendigung des Zutropfens wird 3 Stunden bei 25 bis 30 °C nachgerührt. Die organische Phase wird nachfolgend auf 10 kg Eis geschüttet, mit Natriumbicarbonatlösung neutral gewaschen und nochmals mit Wasser nachgewaschen. Die organische Phase wird abgetrennt und von verbliebenden Wasseranteilen durch Zentrifugieren weitgehend befreit. Das so erhaltene, flüssige Dinitroalkylbenzolgemisch wurde ohne weitere Reinigung der nächsten Reaktionsstufe zugeführt.

Ausbeute : 2,7 kg ; $NO_2$-Gehalt : 27,5 % (Theorie : 27,4 %).

b) Hydrierung des Dinitroalkylbenzolgemisches :

In einem Rührautoklaven werden 672 g der Dinitro-verbindung gemäß a) in 1 700 ml Ethanol gelöst und mit 70 g Raney-Nickel versetzt. Bei 40 °C und 20 bis 40 bar Wasserstoffdruck wird bis zum Ende der Wasserstoffaufnahme gerührt. Anschließend wird entspannt, vom Katalysator abfiltriert und das Ethanol abdestilliert. Man erhält 550 g Rohamingemisch, welches als solches oder auch nach destillativer Reinigung für die erfindungsgemäße Verwendung geeignet ist. Das Rohamingemisch weist einen Stickstoffgehalt von 10,06 % (Theorie : 10,1 %) auf.

200 g Rohamingemisch werden unter vermindertem Druck destilliert. Auf diese Weise können 167 g Diamingemisch als bei 185 bis 204 °C/2,1 mbar siedende Fraktion erhalten werden (Ausbeute : 83,5 %).

Analyse (%) :

| | C | H | N |
|---|---|---|---|
| Gefunden : | 78,6 | 11,5 | 10,0 |
| Theorie : (bezogen auf $C_{18}H_{32}N_2$) | 78,3 | 11,6 | 10,1 |

Diisocyanatgemisch I :

In einem 6 l Vierhalskolben, mit Rührer, Thermometer, Gaseinleitungsrohr und Rückflußkühler versehen, werden 3 l trockenes Chlorbenzol vorgelegt. Unter Rühren und Kühlung (– 10 °C) werden ca. 800 g Phosgen einkondensiert, anschließend läßt man unter Kühlung bei – 10 bis – 5 °C 550 g des bei der Herstellung von Diamingemisch I beschriebenen Rohamingemischs, in 500 ml Chlorbenzol gelöst, zutropfen. Unter weiterem Einleiten von Phosgen erwärmt sich die Lösung anschließend ohne weitere Kühlung auf ca. 30 °C. Nach Abklingen der Wärmetönung wird langsam bis 100 °C aufgeheizt und bis zum Ende der Chlorwasserstoffentwicklung Phosgen eingeleitet (insgesamt 970 g). Das überschüssige Phosgen wird mit Stickstoff ausgeblasen und die Lösung im Vakuum eingedampft. Man erhält 638 g Rohisocyanatgemisch mit einem NCO-Gehalt von 25 % (Theorie : 25,6 %). Das so erhaltene Diisocyanat kann ohne weitere Reinigung beim erfindungsgemäßen Verfahren eingesetzt werden, oder aber einer zusätzlichen destillativen Reinigung unterworfen werden :

300 g des Diisocyanatgemisches werden unter vermindertem Druck destilliert. Bei einem Druck von 2,2 mbar destillieren innerhalb des Temperaturbereichs von 185 bis 203 °C 273 g eines praktisch farblosen Gemischs von Diisocyanaten der Formel

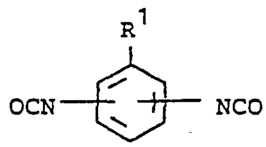 ($R^1$ = $C_{10}$-$C_{13}$-Alkyl)

welches auch bei Abkühlung auf – 50 °C keinerlei Tendenz zur Kristallbildung erkennen läßt.

Analyse (%) :

| | NCO | C | H | N |
|---|---|---|---|---|
| Gefunden : | 25,5 | 73,6 | 8,6 | 8,4 |
| Theorie : (bezogen auf $C_{20}H_{28}N_2O_2$) | 25,6 | 73,2 | 8,5 | 8,5 |

Nach Kernresonanzmessungen sind die Isocyanatgruppen des Diisocyanats in meta-Stellung zueinander angeordnet. Die Hauptkomponente des Gemischs besteht aus 1-Alkyl-2,4-diisocyanatobenzol.

## Beispiel 1

In einem Dreihalskolben mit Rührer, Tropftrichter, Innenthermometer und Blasenzähler werden unter Stickstoff 1 000 g eines Diisocyanatgemisches, bestehend aus 80 % 2,4- und 20 % 2,6-Diisocyanatotoluol, 1 g Toluolsulfonsäureethylester und 0,1 g Zinkacetylacetonat vorgelegt und auf 80 °C erwärmt. Anschließend läßt man 83 g des Diamingemisches I vom Siedepunkt 185 bis 204 °C/2,1 mbar unter Rühren bei 80 °C während 1 Stunde zutropfen. Dann wird während 2 Stunden bei 100 °C nachgerührt und schließlich 1,5 ml Benzoylchlorid als Stabilisator hinzugefügt. Nach Abkühlen auf Raumtemperatur liegt ein weitgehend Biuret-modifiziertes Polyisocyanat mit einem NCO-Gehalt von 41,3 % vor. Während der Herstellung des modifizierten Polyisocyanats traten zu keinem Zeitpunkt Ausfällungen von unlöslichen Produkten auf.

## Beispiel 2

Entsprechend Beispiel 1 werden 2 700 g Diisocyanatgemisch, bestehend aus 80 % 2,4- und 20 % 2,6-Diisocyanatotoluol, 2,7 g Toluolsulfonsäureethylester und 0,3 g Zinkacetylacetonat auf 80 °C erwärmt und mit 280 g Diamingemisch I zur Reaktion gebracht. Man läßt Stunden bei 100 °C nachrühren, stabilisiert mit 2 g Benzoylchlorid und erhält ein im wesentlichen Biuret-modifiziertes Polyisocyanat mit einem NCO-Gehalt von 38,8 %. Das Auftreten fester Bestandteile konnte zu keinem Zeitpunkt beobachtet werden.

## Beispiel 3

1 000 g eines Diisocyanatgemisches, bestehend aus 55 % 2,4'- und 45 % 4,4'-Diisocyanatodiphenylmethan, 1 g Toluolsulfonsäureethylester und 0,2 g Zinkacetylacetonat werden auf 80 °C erwärmt und mit 55 g des Diamingemischs I innerhalb von 1 Stunde versetzt. Man läßt 3 Stunden bei 100 °C

nachrühren, stabilisiert mit 1 g Benzoylchlorid und erhält ein Feststoff-freies, weitgehend Biuret-modifiziertes Polyisocyanat mit einem NCO-Gehalt von 29,0 %.

Herstellung von Harnstoffpolyisocyanaten

Beispiel 4

945 g Diisocyanatgemisch, bestehend aus 80 % 2,4- und 20 % 2,6-Diisocyanatotoluol werden bei 30 bis 40 °C mit 55 g des Diamingemischs I innerhalb von 1 Stunde versetzt. Man läßt 5 Stunden bei 50 °C nachrühren, stabilisiert mit 1 g Benzoylchlorid und erhält ein Harnstoffgruppen enthaltendes flüssiges Polyisocyanat mit einem NCO-Gehalt von 44,2 %. Aus dem Infrarotspektrum geht hervor, daß keine biurethaltigen Beimengen vorlagen (fehlende Absorption im Bereich von ca. 1 700 $cm^{-1}$).

Herstellung von Harnstoff- und Biuretgruppen enthaltenden Polyisocyanaten

Beispiel 5

Der Ansatz von Beispiel 4 wurde wiederholt mit der Maßgabe, daß 4 Stunden bei 140 °C nachgerührt wurde. Man erhielt ein Harnstoff- und Biuretgruppen-enthaltendes flüssiges Polyisocyanat mit einem NCO-Gehalt von 43,7 %.

Beispiel 6

Der Ansatz von Beispiel 4 wurde nach einem erfindungsgemäß bevorzugten Verfahren wiederholt, indem die Reaktion unter Mitverwendung von 1 g Toluolsulfonsäureethylester und 0,2 g Zinkacetylacetonat durchgeführt wurde, mit anschließendem 4-stündigem Nachrühren bei 100 °C. Die mit 1 g Benzoylchlorid stabilisierte Lösung wies einen NCO-Gehalt von 43,0 % auf ; ein Beweis dafür, daß die gemäß Beispiel 4 zu erzeugenden Harnstoffgruppen nach der Verfahrensweise dieses Beispiels weitgehend biuretisiert wurden.

Beispiel 7

860 g Diisocyanatgemisch I, 1 g Toluolsulfonsäureethylester und 0,2 g Zinkacetylacetonat werden auf 80 °C erwärmt und mit 140 g des Diamingemischs I innerhalb von 1 Stunde versetzt. Man läßt 4 Stunden bei 110 °C nachrühren, stabilisiert mit 1 g Benzoylchlorid und erhält ein Feststoff-freies, viskoses, Biuret-modifiziertes Polyisocyanat mit einem NCO-Gehalt von 15,1 %.

Beispiel 8

820 g Diisocyanatgemisch I, 1 g Toluolsulfonsäureethylester und 0,2 g Zinkacetylazetonat werden auf 100 °C erhitzt und portionsweise innerhalb von 1 Std. mit 12,2 g 2,4-Diaminotoluol zur Reaktion gebracht, wobei die Temperatur langsam auf 140 °C gesteigert wird. Man läßt 4 Std. bei 140 °C nachrühren, stabilisiert mit 1 g Benzoylchlorid und erhält ein dünnflüssiges, Biuret-modifiziertes Polyisocyanat mit einem NCO-Gehalt von 20,1 %.

Beispiel 9

870 g Diisocyanatgemisch, bestehend aus 80 % 2,4- und 20 % 2,6-Diisocyanatoluol, 1 g Toluolsulfonsäureethylester und 0,1 g Zinkacetylacetonat werden auf 120 °C erhitzt. Innerhalb von 1 Std. läßt man eine auf 100 °C erhitzte Lösung von 18 g 2,4-Diaminotoluol in 14 g Diamingemisch I zutropfen, rührt 3 Std. bei 140 °C nach und stabilisiert mit 1 g Benzoylchlorid. Das so erhaltene, dünnflüssige, Biuretmodifizierte Polyisocyanat weist einen NCO-Gehalt von 44,5 % auf.

Beispiel 10

330 g Diisocyanatgemisch I, 525 g Diisocyanatgemisch, bestehend aus 80 % 2,4- und 20 % 2,6-Diisocyanatotoluol, 1 g Toluolsulfonsäureethylester und 0,2 g Zinkacetylacetonat werden auf 130 °C erhitzt. Innerhalb von 1 Std. trägt man 12 g 2,4-Diaminotoluol ein, rührt 3 Std. bei 140 °C nach und erhält nach Stabilisierung mit 1 g Benzoylchlorid ein dünnflüssiges, Biuret-modifiziertes Polyisocyanat mit einem NCO-Gehalt von 38,0 %.

Beispiel 11

Beispiel 10 wird wiederholt, indem eine auf 100 °C erhitzte Lösung von 18 g 2,4-Diaminotoluol in 14 g

Diamingemisch I anstatt 12 g 2,4-Diaminotoluol eingetropft wird. Nach entsprechender Weiterverarbeitung erhält man ein dünnflüssiges, Biuret-modifiziertes Polyisocyanat mit einem NCO-Gehalt von 36,2 %.

Beispiel 12

1 000 g eines Diisocyanatgemisches, bestehend aus 80 % 2,4- und 20 % 2,6-Diisocyanatotoluol, 1 g Toluolsulfonsäureethylester und 0,1 g Zinkacetylazetonat werden auf 100 °C erhitzt. Anschließend läßt man eine Lösung von 10 g 4,4'-Diaminodiphenylmethan, in 42 g Diamingemisch I gelöst, innerhalb von 1 Std. zutropfen. Man rührt 3 Std. bei 130 °C nach, stabilisert mit 0,5 g Benzoylchlorid und erhält ein dünnflüssiges Biuretmodifiziertes Polyisocyanat mit einem NCO-Gehalt von 40,9 %.

Beispiel 13

Herstellung eines Schaumstoffs

Werden 46,1 Gew.-Teile des Biuret-modifizierten Polyisocyanats gemäß Beispiel 1 (NCO : 41,3 %) mit einem vorab hergestellten Gemisch aus 100 Gew.-Teilen eines trifunktionellen Polyetherpolyols der OH-Zahl 28, welches durch Propoxylierung von Trimethylolpropan und anschließende Ethoxylierung des Propoxylierungsprodukts (insgesamt 13 Gew.-% Ethylenoxid) erhalten worden ist, 3,0 Gew.-Teilen Wasser, 0,5 Gew.-Teilen Polysiloxanstabilisator, 2,0 Gew.-Teilen Diethanolamin, 0,6 Gew.-Teilen einer 33 %igen Lösung von Diethylentriamin in Dipropylenglykol innig vermischt, so entsteht unter Aufschäumen und Verfestigung ein hochelastischer Weichschaum dessen Kenngrößen in Tabelle 1 zusammengefaßt sind.

Vergleichsbeispiel 13a

Beispiel 13 wird wiederholt, jedoch verwendet man anstelle des erfindungsgemäßen Biuretpolyisocyanats 46,9 Gew.-Teile eines handelsüblichen Allophanat-modifizierten Polyisocyanats auf Basis eines Diisocyanatgemischs, bestehend aus 80 % 2,4- und 20 % 2,6-Diisocyanatotoluol mit einem NCO-Gehalt von 40,5 %. Es handelt sich hierbei um ein üblicherweise zur Herstellung von Polyurethan-Weichschaumstoffen eingestzten Polyisocyanat. Die Eigenschaften der jeweils erhaltenen hochelastischen Weichschaumstoffe werden in nachfolgender Tabelle 1 wiedergegeben.

Tabelle 1

| Mechanische Eigenschaften | Beispiel 13 | Vergleichsbeispiel 13a |
|---|---|---|
| Rohdichte (kg/m$^3$) | 35 | 35 |
| Zugfestigkeit (kPa) | 100 | 75 |
| Bruchdehnung (%) | 150 | 100 |
| Stauchhärte (kPa) bei 40 % Verformung | 2,9 | 1,9 |
| Druckverformungsrest (%) bei 90 % Verformung | 10 | 8 |

Beispiel 14

50,2 Gew.-Teile des Biuret-modifizierten Polyisocyanats gemäß Beispiel 2 (NCO : 38,8 %) werden mit einem zuvor hergestellten Gemisch aus

100 Gew.-Teilen eines trifunktionellen Polyetherpolyols der OH-Zahl 35, welches durch Propoxylierung von Trimethylolpropan und anschließende Ethoxylierung des Propoxylierungsprodukts (insgesamt 13 % Ethylenoxid) erhalten worden ist,

| | |
|---|---|
| 3,0 Gew.-Teilen | Wasser |
| 0,5 Gew.-Teilen | eines Polysiloxanstabilisators |
| 2,0 Gew.-Teilen | Diethanolamin |
| 0,2 Gew.-Teilen | Diethylentriamin |
| 0,1 Gew.-Teilen | Dimethylaminoethylether |
| 0,1 Gew.-Teilen | Zinndioktoat |

innig vermischt und in einer offenen Form aufgeschäumt. Es entsteht ein hochelastischer Weichschaum mit folgenden mechanischen Eigenschaften :

| | |
|---|---|
| Rohdichte (kg/m$^3$) | 37 |
| Zugfestigkeit KPa | 90 |
| Bruchdehnung % | 125 |

2. Verwendung der gemäß Anspruch 1 erhaltenen, Harnstoff- und/oder Biuretgruppen aufweisenden Polyisocyanate, gegebenenfalls in einer in überschüssigem Ausgangsdiisocyanat gelösten Form, als Isocyanatkomponente bei der Herstellung von Polyurethankunststoffen nach dem Isocyanat-Polyadditionsverfahren.

**Claims**

1. Process for the preparation of aromatic polyisocyanates containing urea and/or biuret groups, optionally in the form of a mixture of homologues and/or isomers, by the reaction of aromatic diisocyanates which are free from urea and biuret groups with diamines having primary or secondary amino groups and free from urea and biuret groups at 20 to 180 °C, observing an equivalent ratio of isocyanate groups to primary or secondary amino groups of 5 : 1 to 100 : 1, characterised in that at least 25 mol% of the diisocyanates put into the process correspond to the formula

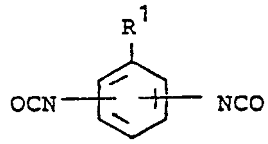

and/or at least 25 mol% of the diamines put into the process correspond to the formula

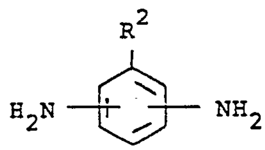

in which formulae,

$R^1$ and $R^2$ represent identical or different radicals and denote in each case saturated aliphatic hydrocarbon radicals having 6 to 18 carbon atoms.

2. Use of the polyisocyanates containing urea and/or biuret groups, obtained according to Claim 1, optionally in the form of solutions in excess starting diisocyanate, as the isocyanate component in the production of polyurethane plastics by the isocyanate polyaddition process.

**Revendications**

1. Procédé de fabrication de polyisocyanates aromatiques éventuellement sous la forme de mélange d'homologues et/ou d'isomères et présentant des groupes urée et/ou biuret, par réaction de diisocyanates aromatiques exempts de groupes urée et biuret avec des diamines exemptes de groupes urée et biuret et contenant des groupes amino primaires ou secondaires, à 20-180 °C, tout en respectant un rapport d'équivalence entre groupes isocyanate et groupes amino primaires ou secondaires de 5 : 1 à 100 : 1, caractérisé en ce qu'au moins 25 moles % des diisocyanates à employer répondent à la formule

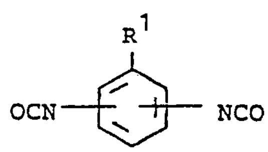

et/ou au moins 25 moles % des diamines à employer répondent à la formule

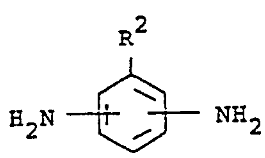

dans ces formules

$R^1$ et $R^2$ représentant des radicaux identiques ou différents et signifiant chaque fois des radicaux hydrocarbonés aliphatiques saturés ayant 6 à 18 atomes de carbone.

2. Utilisation des polyisocyanates obtenus selon la revendication 1, présentant des groupes urée et/ou biuret, éventuellement en une forme dissoute dans le diisocyanate de départ en excès, en tant que composant isocyanate dans la fabrication de matières plastiques en polyuréthanes par le procédé de polyaddition à l'isocyanate.

| | |
|---|---|
| Stauchhärte KPa bei 40 % Verformung | 3,3 |
| Druckverformungsrest % (90 % Verformung) | 10 |

Beispiel 15

Ein Polyisocyanatgemisch, bestehend aus 18,9 Gew.-Teilen des Biuret-modifizierten Polyisocyanats gemäß Beispiel 2 und 25,1 Gew.-Teilen eines Isomeren-Gemischs, bestehend aus 80 % 2,4- und 20 % 2,6-Diisocynatotoluol (NCO-Gehalt der Mischung : 44,2 %), werden mit einem vorab hergestellten Gemisch aus

| | |
|---|---|
| 100 Gew.-Teilen | des trifunktionellen Polyetherpolyols der OH-Zahl 35 aus Beispiel 14, |
| 3,0 Gew.-Teilen | Wasser |
| 0,5 Gew.-Teilen | eines Polysiloxanstabilisators |
| 2,0 Gew.-Teilen | Diethanolamin |
| 0,1 Gew.-Teilen | einer 33 %igen Lösung von Triethylendiamin in Dipropylenglykol |
| 0,1 Gew.-Teilen | Dimethylaminoethylether |

innig vermischt und in einer offenen Form aufgeschäumt. Es entsteht ein hochelastischer Weichschaum, dessen mechanische Eigenschaften in Tabelle 2 zusammengefaßt sind.

Beispiel 16

44,0 Gew.-Teile des harnstoffmodifizierten Polyisocyanates gemäß Beispiel 4 (NCO : 44,2 %) werden mit einem vorab hergestellten Gemisch, wie in Beispiel 15 beschrieben, innig vermischt und in einer offenen Form aufgeschäumt.

Es entsteht ein hochelastischer Weichschaum, dessen mechanische Eigenschaften in Tabelle 2 im Vergleich zu Beispiel 15 (Biuretmodifizierung) dargestellt sind.

Tabelle 2

| | Beispiel 15 | Beispiel 16 |
|---|---|---|
| Rohdichte $kg/m^3$ | 35 | 35 |
| Zugfestigkeit KPa | 90 | 65 |
| Bruchdehnung % | 100 | 90 |
| Stauchhärte KPa bei 40 % Verformung | 2,5 | 2,4 |

**Ansprüche**

1. Verfahren zur Herstellung von gegebenenfalls als Homologen- und/oder Isomerengemisch vorliegenden Harnstoff- und/oder Biuretgruppen aufweisenden aromatischen Polyisocyanaten durch Umsetzung von Harnstoff- und Biuretgruppen-freien aromatischen Diisocyanaten mit Harnstoff- und Biuretgruppenfreien Diaminen mit primären oder sekundären Aminogruppen bei 20 bis 180 °C unter Einhaltung eines Äquivalentverhältnisses zwischen Isocyanatgruppen und primären bzw. sekundären Aminogruppen von 5 : 1 bis 100 : 1, dadurch gekennzeichnet, daß mindestens 25 Mol-% der zum Einsatz gelangenden Diisocyanate der Formel

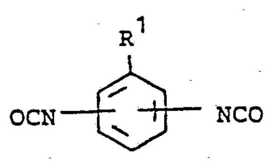

und/oder mindestens 25 Mol-% der zum Einsatz gelangenden Diamine der Formel

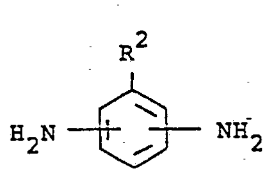

entsprechen, wobei in diesen Formeln

$R^1$ und $R^2$ für gleiche oder verschiedene Reste stehen und jeweils gesättigte aliphatische Kohlenwasserstoffreste mit 6 bis 18 Kohlenstoffatomen bedeuten.